# FASCICULE DE BREVET EUROPEEN

(11) **EP 2 820 003 B1**
(45) Date de publication et mention de la délivrance du brevet: **10.08.2016**
(21) Numéro de dépôt: 13710497.2
(22) Date de dépôt: 21.02.2013
(51) Int. Cl.: C07D 279/18, C07H 21/04, C09B 49/06, C09B 21/00, C07F 9/6547

(54) **DERIVES DU DIAMINOPHENOTHIAZINIUM POUR LE MARQUAGE DE BIOMOLECULES, PROCEDE ET SUPPORT DE MARQUAGE D'OLIGONUCLEOTIDES ET OLIGONUCLEOTIDES OBTENUS**
DIAMINOPHENOTHIAZINDERIVATE ZUR MARKIERUNG VON BIOMOLEKÜLEN, VERFAHREN UND SUBSTRAT ZUR MARKIERUNG VON OLIGONUCLEOTIDEN UND DADURCH GEWONNENE OLIGONUCLEOTIDE
DIAMINOPHENOTHIAZINIUM DERIVATIVES FOR LABELLING BIOMOLECULES, METHOD AND SUBSTRATE FOR LABELLING OLIGONUCLEOTIDES, AND OLIGONUCLEOTIDES OBTAINED

(30) Priorité: 27.02.2012 FR 1251739
(43) Date de publication de la demande: 07.01.2015
(73) Titulaire: Université Claude Bernard Lyon I, 69622 Villeurbanne Cedex (FR); Centre National de la Recherche Scientifique (CNRS), 75794 Paris Cedex 16 (FR)
(72) Inventeur: CHAIX, Carole, F-69970 Chaponnay (FR); DE CROZALS, Gabriel, F-69300 Caluire-et-Cuire (FR)
(74) Mandataire: Sarlin, Laure V.
(86) Numéro de dépôt international: PCT/FR2013/050356
(87) Numéro de publication internationale: WO 2013/128099

(56) Documents cités:
- MAJKEN N. HANSEN ET AL: "Synthesis and Application of a Triazene-Ferrocene Modifier for Immobilization and Characterization of Oligonucleotides at Electrodes", THE JOURNAL OF ORGANIC CHEMISTRY, vol. 75, no. 8, 16 avril 2010 (2010-04-16) , pages 2474-2481, XP055035323, ISSN: 0022-3263, DOI: 10.1021/jo9024368 cité dans la demande
- JUDITH JÄHNCHEN ET AL: "NMR studies on self-complementary oligonucleotides conjugated with methylene blue", BIOPOLYMERS, vol. 79, no. 6, 15 décembre 2005 (2005-12-15), pages 335-343, XP055035324, ISSN: 0006-3525, DOI: 10.1002/bip.20363 cité dans la demande
- UWE MOELLER ET AL: "Versatile Procedure of Multiple Introduction of 8-Aminomethylene Blue into Oligonucleotides", BIOCONJUGATE CHEMISTRY, vol. 6, no. 2, 1 mars 1995 (1995-03-01), pages 174-178, XP055035328, ISSN: 1043-1802, DOI: 10.1021/bc00032a004 cité dans la demande
- F. SCHUBERT ET AL: "Covalent Attachment of Methylene Blue to Oligonucleotides", NUCLEOSIDES AND NUCLEOTIDES, vol. 14, no. 6, 1 août 1995 (1995-08-01), pages 1437-1443, XP009161974, ISSN: 0732-8311, DOI: 10.1080/15257779508010703

## Description

La présente invention concerne le domaine technique des marqueurs de biomolécules. Plus précisément, la présente invention concerne de nouveaux dérivés de diaminophénothiazinium de la même famille que le bleu de méthylène, et de structure adaptée pour pouvoir être incorporés dans des oligonucléotides, directement lors de leur synthèse. L'invention a également pour objet les procédés de croissance des oligonucléotides utilisant de tels dérivés, les oligonucléotides ainsi obtenus et des supports de synthèse pour oligonucléotides sur lesquels des dérivés de diaminophénothiazinium sont greffés.

Le bleu de méthylène est une molécule aux propriétés multiples. C'est, entre autres, une molécule électroactive qui a la propriété de passer d'un état réduit à un état oxydé sous l'action d'un potentiel d'oxydation, en libérant 2 électrons (Farjami, E.; Clima, L.; Gothelf, K. V.; Ferapontova, E. E. Analyst 2010, 135, 1443-1448). Le bleu de méthylène est également une molécule colorée, qui absorbe à la longueur d'onde de 665 nm en milieu aqueux et une molécule fluorescente, dont la longueur d'onde d'excitation est de 665 nm et celle d'émission est de 682 nm.

Pour toutes ces raisons, le bleu de méthylène est un marqueur biologique très efficace, que ce soit pour réaliser une détection optique ou une détection électrochimique de l'entité biologique marquée.

Dans les années 80 des synthèses en phase solide des biomolécules et notamment des peptides et des oligonucléotides ont été développées, Le procédé chimique actuellement le plus utilisé pour la synthèse des oligonucléotides est connu sous le nom de « la méthode aux phosphoramidites ». Cette méthode consiste à ajouter, d'une manière séquentielle, un seul nucléotide à la fois, à une chaîne croissante d'oligonudéotides. Un groupe protecteur des fonctions hydroxyle dans des conditions de synthèse des oligonucléotides tels, que par exemple, un groupe diméthoxytrityle, est placé à l'extrémité 5' d'une chaîne croissante d'oligonucléotide qui est elle-même accrochée par son extrémité 3' à un support solide. Ce groupe protecteur est éliminé grâce à un traitement acide, par exemple avec de l'acide trichloroacétique, et l'extrémité 5' de l'oligonucléotide ainsi libérée est ensuite couplée avec un synthon nucléotidique substitué à son extrémité 3' avec un dérivé phosphoramidite, ce qui permet d'accroitre la chaine nucléotidique. L'agent d'activation de la réaction de couplage est, par exemple, le tétrazole qui réagit avec la fonction phosphoramidite du synthon, formant in situ un intermédiaire très réactif vis-à-vis de la fonction alcool déprotégée présente à l'extrémité 5' de l'oligonucléotide fixé au support. Il est également possible d'utiliser à la place d'un groupe phosphoramidite, un groupe phosphodiester (Reese C.B., Titmas R.C., Yau L., Tetrahedron Let 1978, 30, 2727-2730; Efimov, V.A., Burgakova, A.A., Dubey, I.Y., Polushin, N.N., Chakhmakhcheva, O.G., Orchinnikov, Y.A., NucL Acids, Res., 1986, 14, 6525-6540) ou hydrogénophosphonate (Froehler, B.C., Matteucci, M.D. Tetrahedron Let. 1986, 27, 469-472). Pour plus de détails sur ces techniques, on pourra se référer à Current Protocols în Nucleic Acid Chemistry, Volume 1, Editors : S. L. Beaucage, D. E. Bergstrom, G, D. Glick, R. A. Jones, John Wiley & Sons, Inc., 2004 ; Protocols for oligonucleotides and analogs, Volume 20, Editor: S. Agrawal, Methods in molecular biology, Humana Press, 1993. Il est intéressant d'introduire à ce stade des synthons nucléotidiques modifiés contenant, par exemple, un marqueur de type fluorescent ou électrochimique.

Dans les synthétiseurs automatiques, la séquence d'une réaction peut être répétée un grand nombre de fois, par exemple, jusqu'à 150 fois. Dès qu'un oligonucléotide de séquence voulue est synthétisé, il est libéré du support par un traitement basique qui permet également d'éliminer les différents groupements protecteurs. L'oligonucléotide obtenu peut alors être purifié par HPLC ou par électrophorèse sur gel.

La chimie de synthèse des oligonucléotides en phase solide nécessite donc une étape finale de déprotection des nucléotides formant l'oligonudéotide et de décrochage du support, en milieu basique, typiquement dans une solution aqueuse de NH₄OH 30% en volume ou de K₂CO₃ à 0,05M. Ces traitements ne sont pas compatibles avec un greffage préalable du bleu de méthylène sur l'oligonucléotide lors de sa synthèse, car le bleu de méthylène est une phénothiazine substituée par deux diméthylamines qui n'est pas stable en milieu basique. En effet, les groupements diméthylamine se clivent rapidement en milieu basique entraînant la perte des propriétés électrochimiques et colorantes (Mills A., Hazafy D., Parkinson J., Tuttle T., Hutchings M. G., Effect of alkali on methylene blue (CI. Basic Blue 9) and other thiazine dyes, Dyes and Pigments 2011, 88, (2), 149-155).

A ce jour, les stratégies proposées pour greffer un dérivé de bleu de méthylène sur une blomolécule du type oligonucléotide font intervenir un couplage post-synthèse sur la biomolécule, c'est-à-dire un couplage lors d'une étape finale sur la biomolécule déjà formée. Un dérivé ester activé du bleu de méthylène a été synthétisé pour être couplé sur une biomolécule porteuse de fonctions amine primaire (Jähnchen J., Purwanto M. G. M., Weisz K., NMR studies on self-complementary oligonucleotides conjugated with methylene blue, Biopolymers 2005, 79, (6), 335-343; Farami, Anal. Chem. 2011; Hansen, JOC, 2010). Les travaux de Rowe et al. (Aaron Rowe, Kelly N Chuh, Arica A Lubin, Erin A Miller, Brett M Cook, Daniel N Hollis, and Kevin W. Plaxco, Anal. Chem, 2011, 83 (24), 9462-9466) prévoient quant à eux de greffer sur un oligonucléotide déjà formé du bleu de méthylène grâce à un lien peptidique ou un lien hydrazone. Un dérivé 8-amino bleu de méthylène a également été décrit pour effectuer un couplage sur un polymère porteur de fonctions acide (Uwe Moller, Frank Schubert, and Dieter Cech, Bioconjugate, 1995, 6, 174-178),

D'une manière générale, il a été considéré dans l'art antérieur que le bleu de méthylène devait être couplé en toute dernière étape de la synthèse, pour limiter les risques de dégradation rencontrés dans des milieux trop agressifs utilisant une base ou un agent réducteur utilisé dans les synthèses de biomolécules, et notamment dans le cas de la synthèse des oligonucléotides.

Dans le cadre de l'invention, les inventeurs proposent des nouveaux dérivés de diaminophénothiazinium qui peuvent être directement intégrés dans des biomolécules, et en particulier dans des oligonucléotides lors de leur synthèse.

Dans ce contexte, la présente invention concerne des dérivés de diaminophénothiazinium de formule (1) : 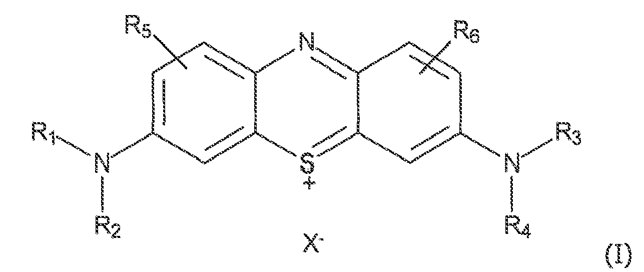 dans laquelle :
- un des groupes R₁, R₂, R₃ et R₄, représente -A₁-OR₇, avec A₁ qui représente une chaîne alkylène linéaire ou ramifiée comprenant de 2 à 12 carbones, les atomes d'oxygène et d'azote étant séparés par au moins deux atomes de carbone consécutifs et R₇ qui représente un groupe phosphoré capable de réagir avec un hydroxyle libre dans des conditions de synthèse des oligonucléotides, ledit groupe formant avec OA₁ auquel il est lié un groupe phosphoramidite, phosphodiester ou hydrogénophosphonate,
- les autres groupes R₁, R₂, R₃ et R₄, identiques ou différents, représentent indépendamment l'un de l'autre :
   o un groupe -Ay₂₋OR₈, avec A₂ qui représente une chaîne alkylène linéaire ou ramifiée comprenant de 2 à 12 carbones, les atomes d'oxygène et d'azote étant séparés par au moins deux atomes de carbone consécutifs, et R₈ qui représente un groupe protecteur des fonctions hydroxyle dans des conditions de synthèse des oligonucléotides choisi parmi les groupes trityle, 4-O-monométhoxytrityle, 4,4'-O-diméthoxytrityle, tert-butyldiméthyisilyle, acétyle, trifluoroacétyle, 9-phénylxanthen-9-yle et fluorénylméthyloxycarbonyle,
   o un groupe alkyle de 2 à 12 atomes de carbone,
   o ou bien R₁ et R₂ ou R₃ et R₄ sont liés entre eux pour former avec l'atome d'azote auquel ils sont liés un groupe pipéridinyle ou pyrrolidinyl,
- R₅ et R₆ identiques ou différents, représentent, indépendamment l'un de l'autre, un atome d'hydrogène, de chlore, brome, iode ou fluor, ou un groupe alkyle de 1 à 12 atomes de carbone, un groupe alcényle de 2 à 12 atomes de carbone, un groupe alcynyle de 2 à 12 atomes de carbone, un groupe acyle ou un groupe phényle,
- X⁻ représente un anion, de préférence choisi parmi Cl⁻, I⁻, ClO₄⁻, NO₃⁻.

Dans le cadre de l'invention, les groupements diméthylamine du bleu de méthylène ont été remplacés par des chaînes plus longues et plus encombrées, afin d'obtenir un dérivé de bleu de méthylène qui une fois intégré à un oligonucléotide, est plus stable, notamment en conditions basiques. Un tel dérivé est donc compatible avec une utilisation dans la synthèse des oligonucléotides supportées qui nécessitent en étape finale un traitement basique pour libérer l'oligonucléotide formé du support de synthèse et/ou déprotéger les nucléotides.

De plus, il a également été constaté que ces modifications chimiques n'affectaient pas, de manière problématique, les propriétés oxydo-réductrices, des dérivés de diaminophénothiazinium ainsi obtenus, confirmant ainsi leur intérêt pour le marquage des oligonucléotides, voire d'autres biomolécules telles que les peptides.

Selon des modes de réalisation préférés, notamment en termes de stabilité, A₁ et A₂ (lorsqu'il est présent) sont des chaînes alkylène linéaires ou ramifiées, dans lesquelles de 2 à 6 atomes de carbone consécutifs séparent les atomes d'oxygène et d'azote.

Dans le cadre de l'invention, il est possible que trois des groupes R₁, R₂, R₃ et R₄ représentent un groupe -A₂-OR₈ tel que défini dans l'invention, Dans ce cas, il est possible de greffer plusieurs nucléotides sur un même dérivé de diaminophénothiazinium, ce qui permet de synthétiser des oligonucléotides ramifiés. Selon d'autres modes de réalisation, au moins un des groupes R₁, R₂, R₃ et R₄, ne représente pas un groupe -A₂-OR₈ et le ou lesdits groupes Ri, R₂, R₃ ou R₄ différent(s) de -A₁-OR₇ et de -A₂-OR₈ représente(nt) un groupe alkyle de 2 à 12 atomes de carbone, de préférence de 4 à 12 atomes de carbone. Dans ce cas, les dérivés de diaminophénothiazinium peuvent répondre à l'une des formules données ci-après :
- formule (Ia) : 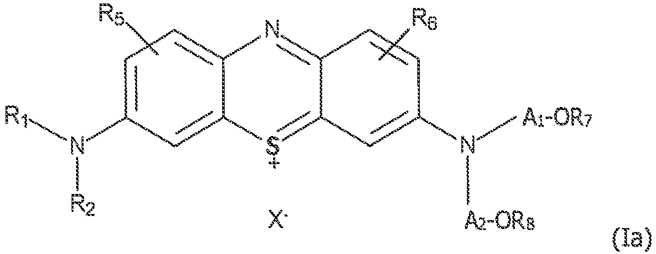 dans laquelle :
   - A₁, A₂, R₅, R₆, R₇, R₈ et X⁻ sont tels que définis pour les composés de formule (I),
   - R₁ et R₂, identiques ou différents, représentent indépendamment l'un de l'autre un groupe alkyle de 2 à 12 atomes de carbone, de préférence de 4 à 12 atomes de carbone, ou R₁ et R₂ sont liés entre eux pour former avec l'atome d'azote auquel ils sont liés un groupe pipéridinyle ou pyrrolidinyle;
- formule (Ib) : 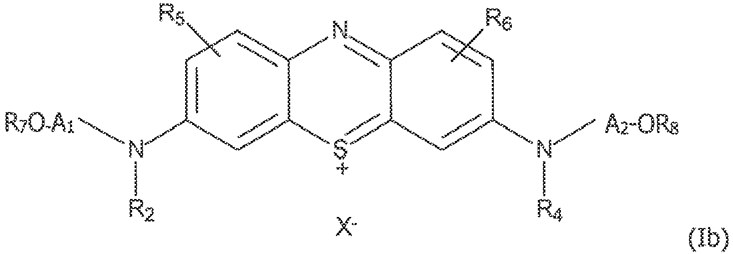 dans laquelle :
   - A₁, A₂, R₅, R₆, R₇, R₈ et X⁻ sont tels que définis pour les composés de formule (I),
   - R₂ et R₄, identiques ou différents, représentent indépendamment l'un de l'autre un groupe alkyle de 2 à 12 atomes de carbone, de préférence de 4 à 12 atomes de carbone ; et
- formule (Ic) : 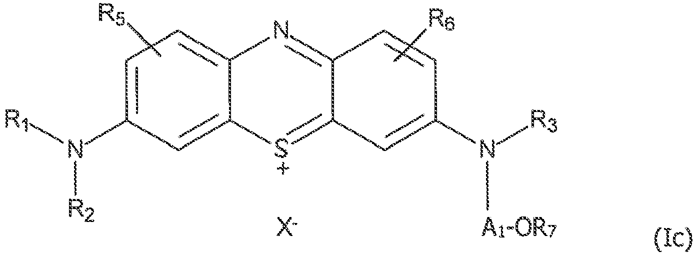 dans laquelle :
   - A₁, R₅, R₆, R₇ et X⁻ sont tels que définis pour les composés (I),
   - R₁ et R₂, identiques ou différents, représentent indépendamment l'un de l'autre un groupe alkyle de 2 à 12 atomes de carbone, de préférence de 4 à 12 atomes de carbone, ou R₁ et R₂ sont liés entre eux pour former avec l'atome d'azote auquel ils sont liés un groupe pipéridinyle ou pyrrolidinyle,
   - R₃ représente un groupe alkyle de 2 à 12 atomes de carbone, de préférence de 4 à 12 atomes de carbone.

Quel que soit le composé de formule (I), (Ia), (Ib) ou (Ic) précédemment décrit, R₅ et R₆ représentent, par exemple, un atome d'hydrogène, comme dans le cas du bleu de méthylène.

Le groupe R₇ peut être du type -P(NR'aR'b)R'c (et forme donc un phosphoramidite avec -OA₁-), -P(O)(OH)(OR'd) (et forme donc un phosphodiester avec -OA₁-) ou -P(O)(OH)H (et forme donc un hydrogénophosphonate avec "OA₁-), dans lesquels R'a, R'b, R'c et R'd sont, par exemple, des groupe alkyle ou phényle éventuellement substitué. De manière avantageuse, dans les composés de formule (I), (Ia), (Ib) et (Ic) précédemment décrits, un des groupes R₁, R₂, R₃ et R₄ représente un groupe -A₁-OR₇ dans lequel R₇ représente un groupe -P[N(ⁱPr)₂](OCH₂CH₂C≡N) (avec ⁱPr = isopropyle) et forme donc un phosphoramidite avec OA₁ et A₁ est tel que précédemment défini, R₇ peut plus généralement représenter un groupe -P{N[(C₂-C₁₂)alky]₂}(OCH₂CH₂C≡N), R₇ peut également représenter un groupe tel que le *O-*(2-chlorophénylphosphate) ou le *O*-2-(1-méthylimidazol-2-yl)phénylphosphate, de manière à former un phosphodiester avec OA₁, R₇ peut également représenter un groupe -P(O)(OH)H,

De tels dérivés de diaminophénothiazinium pourront être préparés selon des techniques bien connues de l'homme de l'art, notamment par analogie avec les techniques décrites dans les exemples. Pour l'essentiel, il est possible de former des dérivés aminés de phénothiazinium par réaction, notamment à température ambiante, d'un sel de phénothiazinium, tel que le tétraiodure de phénothiazinium, avec une dialkylamine, une dihydroxyalkylamine ou une alkyl(hydroxyalkyl)amine dans un alcool tel que le méthanol ou un solvant chloré tel que le dichlorométhane,

Le rapport molaire amine/sel de phénothiazinium sera environ égal à 2 lorsque l'on souhaite greffer une seule amine sur le cycle et sera supérieur à 2 lorsque l'on souhaite greffer deux amines identiques de part et d'autre du cycle phénothiazinium (cas des composés de formule (Ib) dans lesquels A₁=A₂ et R₂=R₄). Pour les composés dans lesquels il est nécessaire de greffer deux amines différentes de part et d'autre du cycle phénothiazinium (cas des composés (Ia) et des composés (Ib) dans lesquels A₁ est différent de A₂ et/ou R₂ est différent de R₄), une nouvelle réaction sera réalisée avec une autre amine dans des conditions analogues. Au moins une des amines utilisées est substituée par un groupe hydroxyalkyle. Un couplage est ensuite réalisé sur la fonction hydroxyle avec au moins un dérivé halogéné, et en particulier un dérivé chloré Cl-R₇ ou Cl-R₈, pour obtenir la fonction -O-R₇ ou -O-R₈ désiré. Un tel couplage est réalisé dans des conditions bien connues de l'homme de l'art, notamment en présence de diisopropyléthylamine dans l'acétonitrile. Deux couplages successifs pourront être réalisés pour obtenir deux fonctionnalisations différentes, dans le cas où au moins deux fonctions hydroxyle sont présentes,

Les dérivés selon invention pourront être intégrés dans un oligonucléotide, dans un procédé de synthèse supportée classique, à la place d'un nucléotide.

Le terme « oligonucléotide » désigne un enchaînement d'au moins 2 nucléotides (désoxyribonucléotides ou ribonucléotides, ou les deux), naturels ou modifiés, susceptibles de s'hybrider, dans des conditions appropriées d'hybridation, avec un oligonucléotide au moins partiellement complémentaire. Par «nucléotides», on entend un composé organique consistant en une base purine ou pyrimidine liée à un ose (ribose ou deoxyribose) et à un groupe phosphate. Par « nucléotide modifié », on entend, par exemple, un nucléotide comportant une base modifiée et/ou comportant une modification au niveau de la liaison internucléotidique et/ou au niveau du squelette. A titre d'exemple de base modifiée, on peut citer l'inosine, la méthyl-5-désoxycytidine, la diméthylamino-5-désoxyuridine, la diamino-2, 6-purine et la bromo-5-désoxyuridine. Pour illustrer une liaison internucléotidique modifiée, on peut mentionner les liaisons phosphorothioate, N-alkylphophoramldate, alkylphosphonate et alkylphosphotriester.

La présente invention a également pour objet un procédé de marquage d'un oligonucléotide par un dérivé de diaminophénothiazinium selon l'invention, qui comprend la croissance d'un oligonucléotide greffé sur un support solide, et le remplacement d'un ou plusieurs nucléotides par un ou plusieurs desdits dérivés de diaminophénothiazinium selon l'invention, avant que l'oligonucléotide ne soit détaché du support solide. Au moins une substitution par un dérivé de diaminophénothiazinium peut être effectuée en positions 3' ou 5', sur le premier ou le dernier nucléotide, respectivement. Il est également possible de réaliser l'insertion d'un dérivé de diaminophénothiazinium à l'intérieur de la chaine nucléotidique, le remplacement d'un synthon nucléotidique phosphoramidite, phosphodiester ou hydrogénophosphonate par un dérivé de diaminophénothiazinium selon l'invention, étant alors réalisée avant la fin de la croissance de l'oligonucléotide. Dans le cadre de l'invention, il est possible insérer le dérivé de diaminophénothiazinium à n'importe quelle place sur la chaine nucléotidique, étant donné que ce dernier peut être introduit directement lors de la croissance des oligonucléotides. Il est possible d'incorporer un dérivé de diaminophénothiazinium selon l'invention sur n'importe quelle position d'une séquence oligonucléotidique, De plus, il est possible incorporer un nombre important de dérivés de diaminophénothiazinium au sein d'une séquence oligonucléotidique, par synthèse supportée. Selon un mode de réalisation particulier, au moins deux insertions, par exemple consécutives, sont effectuées, Il convient de noter qu'un tel processus de marquage était déjà décrit dans la demande WO 03/068787 dans le cas de dérivé ferrocène, Néanmoins, le remplacement d'un marqueur du type ferrocène par un dérivé de diaminophénothiazinium va pouvoir apporter une amélioration notable, en termes de performance du marquage. En effet, les marqueurs selon l'invention transfèrent deux électrons par vague d'oxydation, au lieu d'un électron transféré dans le cas du ferrocène. Les dérivés de diaminophénothiazinium sont également plus stables vis-à-vis des conditions oxydantes et plus sensibles à leur environnement stérique et ionique, dans le milieu.

La présente invention a également pour objet les oligonucléotides marqués susceptibles d'être obtenus par le procédé de marquage selon l'invention ou à partir des supports décrits ci-après.

Les dérivés de diaminophénothiazinium selon l'invention sont très réactifs et peuvent aussi être utilisés pour réagir efficacement avec un autre type de biomolécule, polymère ou un support plan ou particulaire, porteur de fonctions alcool ou amine notamment.

L'invention concerne également les supports de synthèse d'oligonucléotides, comprenant au moins un dérivé de diaminophénothiazinium greffé de manière covalente en surface du support et répondant à la formule : 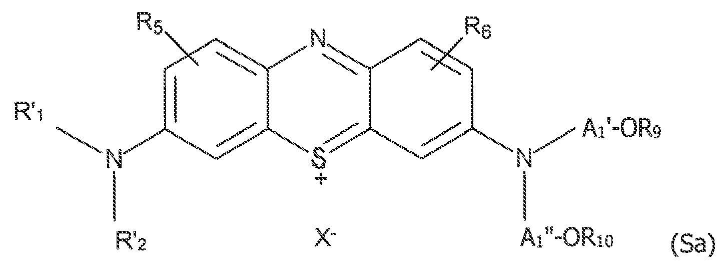 dans laquelle :
- R₅, R₆ et X⁻ sont tels que définis pour les composés de formule (I),
- R₁' et R'₂, identiques ou différents, représentent indépendamment l'un de l'autre un groupe alkyle de 2 à 12 atomes de carbone, de préférence de 4 à 12 atomes de carbone, ou R₁ et R₂ sont liés entre eux pour former avec l'atome d'azote auquel ils sont liés un groupe pipéridinyle ou pyrrolidinyle,
- A₁' et A₁", identiques ou différents, représentent, indépendamment l'un de l'autre, une chaine A₁ telle que définie pour les composés de formule (I), et
- R₉ représente un groupe protecteur des fonctions hydroxyle dans les conditions classiquement utilisées dans la synthèse des oligonucléotides choisi parmi les groupes trityle, 4-0-monométhoxytrityle, 4,4'-O-diméthoxytrityle, tert-butyldiméthylsilyle, acétyle, trifluoroacétyle, 9-phénylxanthen-9-yle et fluorénylméthyloxycarbonyle; et
- R₁₀ représente -CO-A₃-CO-NH-Support, avec A₃ qui représente une chaîne alkylène linéaire ou ramifiée comprenant de 1 à 6 atomes de carbone ; ou 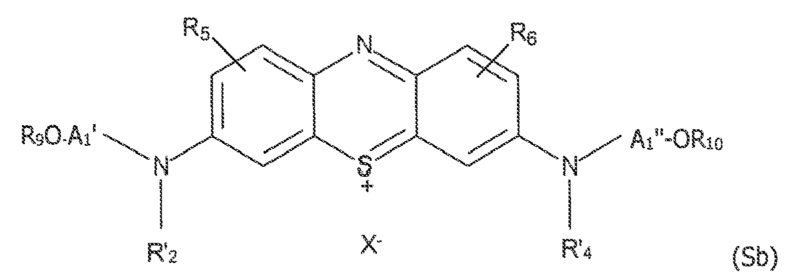
dans laquelle :
- R₅, R₆ et X⁻ sont tels que définis pour les composés de formule (I),
- R'₂ et R'₄, identiques ou différents, représentent indépendamment l'un de l'autre un groupe alkyle de 2 à 12 atomes de carbone, de préférence de 4 à 12 atomes de carbone,
- A₁' et A₁", identiques ou différents, représentent, indépendamment l'un de l'autre, une chaine A₁ telle que définie pour les composés de formule (I), et
- R₉ représente un groupe protecteur des fonctions hydroxyle dans les conditions classiquement utilisées dans la synthèse des oligonucléotides choisi parmi les groupes trityle, 4-0-monométhoxytrityle, 4,4'-O-diméthoxytrityle, tert-butyldiméthylsilyle, acétyle, trifluoroacétyle, 9-phénylxanthen-9-yle et fiuorénylméthyloxycarbonyle ; et
- R₁₀ représente -CO-A₃-CO-NH-Support, avec A₃ qui représente une chaîne alkylène linéaire ou ramifiée comprenant de 1 à 6 atomes de carbone.

Le support peut notamment être choisi parmi les résines, en particulier parmi les résines à base de polystyrène, polyacrylamide, polyéthylèneglycol, cellulose, polyéthylène, polyester, latex, polyamide, polydiméthylacrylamide, les polymères hydrophiles synthétiques ou naturels, les verres et silices à porosité contrôlée, les billes de verre, les gels de silice. Ce type de support comporte généralement des fonctions amine de surface qui vont être modifiées pour faire apparaître des fonctions acide de surface qui vont alors pouvoir être couplées à des fonctions hydroxyalkylamino portées par l'hétérocycle pour conduire aux supports (Sa) et (Sb) ci-dessus.

Certaines définitions utilisées dans la description de l'invention sont rappelées.

Par « alkyle », on entend un groupe hydrocarboné monovalent saturé, linéaire ou ramifié. A titre d'exemples de groupe alkyle, on pourra citer les groupes méthyle, éthyle, n-propyle, isopropyle, n-butyle, isobutyle, sec-butyle, t-butyle, n-pentyle, n-hexyle.

Par « alcényle », on entend un groupe hydrocarboné monovalent insaturé comprenant au moins une double liaison, linéaire ou ramifié.

Par « alcynyle », on entend un groupe hydrocarboné monovalent insaturé comprenant au moins une triple liaison, linéaire ou ramifié.

Par « alkylène », on entend un groupe hydrocarboné bivalent saturé, linéaire ou ramifié. A titre d'exemple de groupe alkylène comprenant de 2 à 12 atomes de carbone, on peut citer -(CH₂)₂-, -CH₂-CH(CH₃)-, -(CH₂)₃-, -(CH₂)₄-, -(CH₂)₅-, -(CH₂)₆-, -(CH₂)₇-, -(CH₂)₈-, -(CH₂)₉-, -(CH₂)₁₀-, -(CH₂)₁₁-, -(CH₂)₁₂-, -CH₂-CH(CH₂CH₃)-, -(CH₂)₂-CH(CH₂CH₃)--...

« Les atomes d'oxygène et d'azote étant séparés par au moins deux atomes de carbone consécutifs » signifie qu'une chaine alkylène linéaire comprenant au moins deux atomes de carbone séparent l'atome d'oxygène de l'atome d'azote (on a donc au moins l'enchainement -N-C-C-O-), les autres atomes de carbone, dans le cas d'une chaine alkylène ramifiée comprenant plus de 2 atomes de carbone, pouvant alors se trouver sur les ramifications.

A titre d'exemple de groupe protecteur des fonctions hydroxyle dans des conditions de synthèse des oligonucléotides, est par exemple choisi parmi les groupes trityle, 4-O-monométhoxytrityle, 4,4'-O-diméthoxytrityle, tert-butyldiméthylsilyle, acétyle, trifluoroacétyle, 9-phénylxanthen-9-yle (pixyle) (le groupement protecteur pixyle est décrit notamment dans le document Chattopadhyaya et Reese, Chem. Soc Chem, Comm., 1978, 639-640) et fluorénylméthyloxycarbonyle (Fmoc).

A titre d'exemple de conditions de synthèse des oligonucléotides, on peut notamment se référer à Current Protocols in Nucleic Acid Chemistry, Volume 1, Editors : S. L Beaucage, D. E. Bergstrom, G. D, Glick, R. A. Jones, John Wiley & Sons, Inc., 2004 ; Protocols for oligonucleotides and analogs.

D'une manière générale, un procédé de synthèse d'oligonucléotide sur support solide comprend les étapes suivantes qui correspondent au premier cycle du procédé :
1) Disposer d'un support sur lequel un nucléoside porteur d'une fonction -OH protégée, par exemple par un groupe trityle, 4-O-monométhoxytrityle, 4,4'-O-diméthoxytrityle, tert-butyldiméthylsilyle, acétyle, trifluoroacétyle, 9-phénylxanthen-9-yle ou fiuorénylméthyloxycarbonyle,
2) Déprotéger la fonction hydroxyle, par un traitement acide, par exemple grâce à une solution d'acide trichloroacétique ou acide dichloroacétique, à une concentration de 2 à 3% en masse dans un solvant tel que le dichlorométhane,
3) Ajouter une solution d'un synthon nucléotidique porteur d'une fonction phosphoramidite et d'une fonction hydroxyle protégée, par exemple à une concentration de 0,05 à 0,2 M (=mol/L), en présence d'un agent de couplage, tel que le tétrazole, l'éthylthiotétrazole, le 4,5-dicyanoimidazole, le saccharin 1-méthylimidazole ou le 5-benzylthiotétrazole, par exemple à une concentration de 0,2 à 0,5 M, dans un solvant tel que l'acétonitrile, ,
4) Bloquer les fonctions hydroxyles n'ayant pas réagi avec les fonctions phosphorées réactives, par action d'anhydride acétique ou d'anhydride phénoxyacétique qui correspond à des conditions plus douces, éventuellement en présence de méthylimidazole ou de diméthylaminopyridine, dans un solvant tel que le tétrahydrofurane ou un mélange tétrahydrofurane/pyridine ou tétrahydrofurane/lutidine ;
5) oxydation du phosphore par action d'une solution d'iode, par exemple à une concentration de 0,02 à 0,1 M, par exemple dans un mélange tétrahydrofurane/pyridine/eau, ou par action d'un péroxyde, par exemple le tert-butylhydropéroxyde, le cumène hydropéroxyde, le péroxyde d'hydrogène ou le bis-triméthylsilyl peroxyde, par exemple à une concentration de 1 à 2 M dans un mélange décane/dichlorométhane, ou par action d'un oxydant non-aqueux comme le (10-camphorsulfonyl)-oxaziridine, par exemple à une concentration de 0,1 à 0,5 M dans l'acétonitrile.

De manière classique, les amines des synthons nucléotidiques utilisés sont protégées, afin d'éviter toutes réactions parasites avec les fonctions phosphorées des synthons nucléotidiques mis en réaction au cours de la synthèse de l'oligonucléotide (étape 3). Ces techniques de protection sont bien connues de l'homme du métier. Notamment, dans le cas de l'adénosine, la fonction amine exocyclique pourra être protégée par un groupe phénoxyacétyle, dans le cas de la cytidine, la fonction aminée exocyclique pourra être protégée par un groupe acétyle, dans le cas de la guanosine, la fonction aminée exocyclique pourra être protégée par un groupe isopropylphénoxyacétyle...

Toutes ces étapes peuvent être réalisées à température ambiante (22°C). Les nucléotides sont protégés tout au long de la synthèse nucléotidique, selon des méthodes bien connues de l'homme de l'art,

En général, les étapes 2), 3), 4) et 5) sont suivies d'un rinçage pour éliminer les réactifs n'ayant pas réagi.

Les étapes 2) à 5) sont répétées autant de fois que nécessaire, avec les synthons nucléotidiques sélectionnés qui peuvent varier d'un cycle à l'autre, pour avoir la séquence d'oligonucléotide souhaitée.

Dans le cas où un synthon nucléotidique porteur d'une fonction phosphodiester est utilisé, à l'étape 3), le synthon nucléotidique porteur d'une fonction phosphodiester est couplé en présence de 1-mésitylènesulfonyl-3-nitro-1,2,4-triazole (MSNT) et de 1-méthylimidazole. Il n'y a pas d'étape d'oxydation 5). De plus, en fin de synthèse, préalablement au traitement basique final, un traitement avec une solution de syn-2-nitrobenzaldoxime et de 1,1,3,3-tétraméthylguanidine dans du dioxane aqueux est réalisé, comme par exemple décrit dans le chapitre 4 du livre édité par Gait, M., Oligonucleotide synthesis. A practical approach (1984) IRL Press, Oxford,

Dans le cas où un synthon nucléotidique porteur d'une fonction hydrogénophosphonate est utilisé, à l'étape 3), le synthon nucléotidique porteur d'une fonction hydrogénophosphonate est couplé en présence de chlorure de pivaloyle en solution dans l'acétonitrile. Les étapes 2) à 4) sont répétées autant de fois que nécessaire pour avoir la séquence d'oligonucléotide souhaitée. L'étape d'oxydation 5) est réalisée en toute fin de synthèse.

Ensuite, une étape finale en milieu basique est réalisée, par exemple, soit grâce à une solution basique de soude, d'ammoniaque, ou de carbonate de potassium ou d'une solution de méthylamine et d'ammoniaque ou d'une solution de diisopropylamine et de 8-mercaptoethanol, soit grâce à de l'ammoniac ou de la méthylamine en phase gazeuse. Ce traitement basique permet de déprotéger les nucléotides présents dans l'oligonucléotide formé et, dans le cas de la méthode utilisant des synthons phosphoramidites ou hydrogénophosphonates, permet également le décrochage du support de l'oligonucléotide formé, Dans le cas de la méthode utilisant des synthons phosphodiesters, ce décrochage se produit lors du traitement avec un mélange de syn-2-nitrobenzaldoxime et de 1,1,3,3-tétraméthylguanidine, Plus précisément, on pourra utiliser soit une solution basique de soude par exemple à une concentration de 0,1 à 0,5 M des nucléotides, dans un solvant tel qu'un mélange eau/méthanol, à une température de 15 à 80 °C, soit grâce à une solution d'ammoniaque par exemple à une concentration de 20 à 40% massique, à une température de 15 à 65 °C, soit une solution de carbonate de potassium par exemple à une concentration de 0,02 à 0,1 M, dans un solvant tel que le méthanol, à une température de 15 à 30 °C, soit une solution de méthylamine et d'ammoniaque (AMA) par exemple à une concentration de 10% à 20% massique chacun, dans un solvant tel que l'eau, à une température de 15 à 65 °C, soit une solution de 10% diisopropylamine/0,25 M 6-mercaptoethanol dans un solvant tel que le méthanol, à une température de 45 à 65°C, soit de l'ammoniac ou de la méthylamine en phase gazeuse, par exemple à une température de 15 à 30 °C. De préférence, un traitement basique avec une solution de carbonate de potassium à une concentration de 0,02 à 0,1 M, dans le méthanol, à une température de 15 à 30 °C, est réalisé.

Dans le cadre du procédé selon l'invention, il est possible d'utiliser à l'étape 1) du premier cycle un support (Sa) conforme à invention, ce qui va permettre d'introduire à l'extrémité 3' de l'oligonucléotide synthétisé un dérivé de diaminophénothiazinium.

Il est également possible lors de n'importe quel cycle d'utiliser un dérivé de diaminophénothiazinium (I), et notamment (Ia) ou (Ib) à l'étape 3) à la place du synthon nucléotidique porteur d'une fonction phosphoramidite et d'une fonction hydroxyle protégée, Ceci permet d'intégrer un dérivé de diaminophénothiazinium à l'intérieur de l'oligonucléotide, à la place d'un nucléotide. L'utilisation à l'étape 3) du dernier cycle d'un dérivé de diaminophénothiazinium (Ic) va permettre l'introduction à l'extrémité 3' du groupement diaminophénothiazinium.

Bien entendu, toutes ces possibilités peuvent être combinées pour incorporer différents groupements diaminophénothiazinium au sein de l'oligonucléotide formé.

Les exemples donnés ci-après, en référence aux figures annexées, lustrent l'invention mais n'ont aucun caractère limitatif.
La **Figure 1** montre les potentiels d'oxydo-réduction du bleu de méthylène et d'un composé selon l'invention incorporé à un oligonucléotide conformément à l'exemple 1.
La **Figure 2** montre l'évolution des pourcentages d'absorbance en fonction du temps de différents dérivés de bisaminophénylthiazinium, en milieu basique.

### EXEMPLE 1; SYNTHESE DU DIMETHOXYTRITYL(DIBUTYL-DIETHANOLAMINO) PHETOTHIAZINIUM PHOSPHORAMIDITE

Le diméthoxytrityl(dibutylamino)-(diéthanolamino) phénothiazinium phosphoramidite est préparé conformément au **SCHEMA 1** ci-dessous.

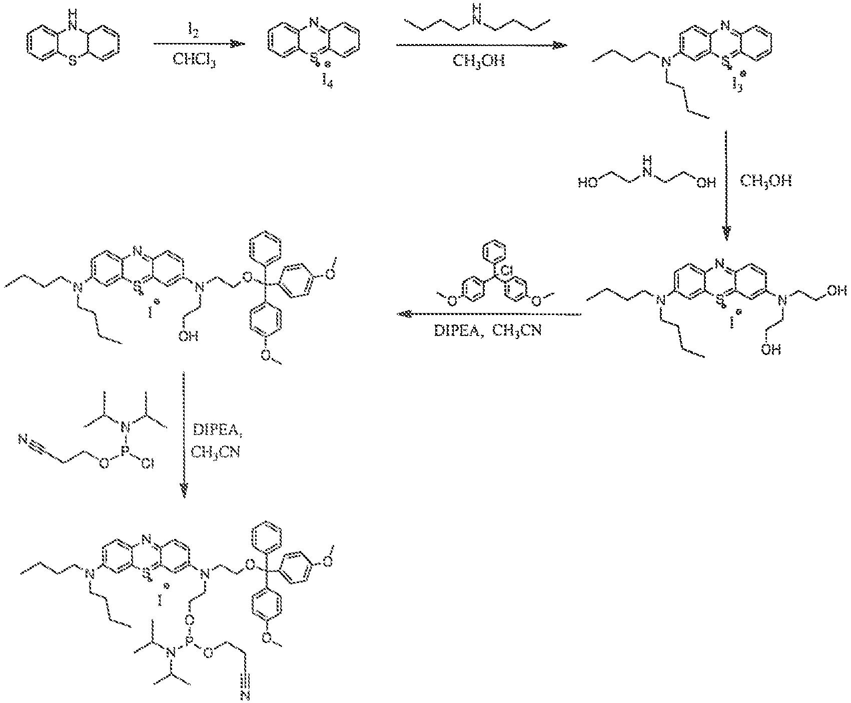

### 1) Préparation du tétraiodure de phénothiazin-5-ium hydrate

Une solution d'iode (15.2 g, 60 mmol) dans le chloroforme (450 mL) est ajoutée goutte à goutte pendant 2h30 à une solution de phénothiazine (4.0 g, 20 mmol) dans le chloroforme (120 mL), dans un ballon de 1L sous agitation magnétique dans un bain de glace. Après la fin de l'addition, le mélange est agité dans un bain de glace pendant toute la nuit.

Le mélange réactionnel est filtré sur verre fritté, Le solide est lavé avec 900mL de chloroforme pour éliminer l'excédent d'iode. Le solide est séché sous pression réduite pendant 2h. Une poudre gris-pourpre est obtenue avec un rendement quantitatif (14.5 g, 20 mmol),
RMN ¹H (DMSO-d6) : δ (ppm) = 8.06 (d, 2H); 7.92 (d, 2H); 7.73 (t, 2H); 7.61 (t, 2H)
SM (ESI+) : masse calculée = 198.0, masse mesurée = 198.0

### 2) Préparation du triiodure de (dibutylamino)phénothiazinium

Le tétraiodure de phénothiazinium (14.5 g, 20 mmol) est dissous dans 290 mL de méthanol à température ambiante. La dibutylamine (6.7 mL, 40 mmol, 2 éq) est ajoutée goutte à goutte à la solution de phénothiazinium, sous agitation magnétique. Le mélange réactionnel est contrôlé par CCM dans un éluant constitué d'un mélange CH₂Cl₂/CH₃OH 95/5 (v/v).

Après 2h de réaction, le mélange est filtré sur verre fritté. Un précipité est récupéré sur le fritté, après lavages avec 3 fois 30 mL de méthanol. Le solide est séché sous pression réduite pendant 1h30. Une poudre gris-pourpre est obtenue avec un rendement de 53% (7.45 g, 10.5 mmol).
RMN ¹H (CD₃CN) : δ (ppm) = 8.25-7.55 (m, 7H, H arom.); 3.85 (m, 4H, 2xN-CH₂); 1.80 (m, 4H, 2xCH₂); 1.50 (m, 4H, 2xCH₂-CH₃); 1,01 (m, 6H, 2xCH₃) SM (ESI+) : masse calculée = 325.2, masse mesurée = 325.2

### 3) Préparation de l'iodure de (dibutylamino)-(diéthanolamino) phénothiazinium

Le iodure de (dibutylamino)phénothiazinium (7,45 g, 10.5 mmol) est dissous dans 150 mL de méthanol à température ambiante (22°C). Une solution de diéthanolamine (2.22 g, 21 mmol, 2 éq) diluée dans le méthanol (35 mL) est préparée puis ajoutée goutte à goutte par une ampoule à brome à la solution de phénothiazinium, sous agitation magnétique à température ambiante. Le mélange réactionnel est contrôlé par CCM dans un éluant constitué d'un mélange CH₂Cl_{2/}CH₃OH 9/1 (v/v).

Après 3h de réaction, le mélange est concentré à l'évaporateur rotatif. Le produit est purifié par chromatographie sur gel de silice (éluant CH₂Cl₂/CH₃OH, 95/5, v/v). Un solide pourpre est obtenu avec un rendement de 49% (2.85 g, 5.13 mmol).
RMN ¹H (CD₃CN) : δ (ppm) = 7.90-7.22 (m, 6H, H arom.); 3.87 (m, 8H, 4xN-CH₂); 3.63 (m, 4H, 2xCH₂-OH); 1.71 (m, 4H, 2xN-CH₂-CH₂); 1.45 (m, 4H, 2xCH₂-CH₃); 1.01 (m, 6H, 2xCH₃)
SM (ESI+) : masse calculée = 428.2, masse mesurée = 428.3

### 4) Préparation de l'iodure de diméthoxytrityl-dibutylamino)-(diéthanolamino) phénothiazinium

Le iodure de (dibutylamino)-(diéthanolamino) phénothiazinium (2.85 g, 5.13 mmol) est introduit dans un ballon de 500 mL préalablement séché à l'étuve. L'acétonitrile anhydre (300 mL) est ajouté sous atmosphère d'argon. La diisopropyléthylamine DIPEA (900 µL, 5.13 mmol, 1 éq) est ajoutée puis une solution de chlorodiméthoxytrityle (1.56 g, 4.62 mmol, 0.9 éq) dans 80 mL d'acétonitrile anhydre est additionnée goutte à goutte à la seringue. Le milieu réactionnel est agité à température ambiante. La réaction est suivie par CCM dans DCM/MeOH/TEA 89/10/1, v/v/v.

Après 1h30, la réaction est arrêtée par l'ajout de 1 mL de méthanol. La solution est concentrée à l'évaporateur rotatif. Le produit est purifié par chromatographie sur gel de silice (éluant CH₂Cl₂/CH₃OH/Et₃N, 94/5/1, v/v/v). Un solide pourpre est obtenu avec un rendement de 52% (2.28 g, 2.66 mmol).
RMN ¹H (CD₃CN) : δ (ppm) = 7.90-6.75 (m, 19H, H arom.); 3.75 (m, 4H, 2xN-CH₂); 3.66 (s, 6H, 2xO-CH₃) ; 3.66-3.45 (m, 8H, 2xN-CH₂ et 2xCH₂-OH); 1.70 (m, 4H, 2xN-CH₂-CH₂); 1.45 (m, 4H, 2xCH₂-CH₃); 1.01 (m, 6H, 2xCH₃) SM (ESI+) : masse calculée = 730.4, masse mesurée = 730.7

### 5) Préparation du iodure de diméthoxytrityl-(dibutylamino)-(diéthanolamino) phénothiazinium phosphoramidite (I.1)

Le iodure de diméthoxytrityl-(dibutylamino)-(diéthanolamino) phénothiazinium (2.28 g, 2.66 mmol) est co-évaporé deux fois dans 20 mL d'acétonitrile anhydre, puis repris dans 50 mL d'acétonitrile anhydre. La diisopropyléthylamine (930 µL, 5.32 mmol, 2 éq) est ajoutée sous atmosphère d'argon, puis la chlorophosphine (710 µL, 3.19 mmol, 1.2 éq) est additionnée goutte à goutte à la seringue. Le milieu réactionnel est agité à température ambiante. La réaction est suivie par CCM dans CH₂Cl₂/CH₃CN/Et₃N 49/49/2, v/v/v.

Après 30 min de réaction, la solution est concentrée à l'évaporateur rotatif. Le produit est purifié par chromatographie sur gel de silice (éluant CH₂Cl₂/ Et₃N, 99/1). Une huile pourpre est obtenue avec un rendement de 26% (743 mg, 0.70 mmol).
RMN ³¹P (CD₃CN) : δ (ppm) = 149.3
SM (ESI+) : masse calculée = 930.5, masse mesurée 930.5

### 6) Synthèse d'un oligonucléotide marqué par le dérivé (dibutylamino)-(diéthanolamino) phénothiazinium

Cet exemple illustre l'incorporation de l'iodure de diméthoxytrityl-(dibutylamino)-(diéthanolamino) phénothiazinium phosphoramidite (composé 1.1) dans la synthèse d'un oligonucléotide de formule : 5'-d(XGGGAAAGGGAGAAGACGTCCAAAAACTTTCCCYY)-3'

Dans cette séquence, A représente l'adénosine, C la cytidine, G la guanosine, T la thymidine, X le (dibutylamino)-(diéthanolamino) phénothiazinium et Y le 1,2-dithiane qui permettra le greffage de l'oligonucléotide sur une surface d'or pour les caractérisations électrochimiques. La synthèse est effectuée en utilisant les synthons phosphoramidites correspondants protégés en position 5' par un groupe diméthoxytrityle. Pour l'adénosine, la fonction aminée exocyclique est protégée par le groupement phénoxyacétyle. Pour la cytidine, la fonction aminée exocyclique est protégée par le groupement acétyle. Pour la guanosine, la fonction aminée exocyclique est protégée par le groupement isopropylphénoxyacétyle.

La synthèse a été effectuée au moyen d'un synthétiseur automatique d'oligonucléotides Applied Biosystems DNA/RNA 394 en utilisant :
- 1 µmole d'une molécule dialcool dithiane (4-0-diméthoxytrityl cyclodithioerythritol) greffée sur un support « Controlled Pore Glass » fonctionnalisée par des chaînes aminées, la liaison entre le 4-O-diméthoxytrityl cyclodithioerythritol et l'amine étant réalisée par un radical succinyl comme décrit dans l'article de P. Liepold, T. Kratzmüller, N. Persike, M. Bandilla, M. Hinz, H. Wieder, H. Hillebrandt, E. Ferrer, G. Hartwich, Anal. Bioanal. Chem. (2008) 391:1759-1772.
- 15 µmoles de (dibutylamino)-(diéthanolamino) phénothiazinium ou de synthons d'adénosine, cytidine, guanosine, thymidine ou dithiane par cycle de synthèse suivant la séquence préalablement programmée,

Dans une première étape, le groupement Diméthoxytrityle de la molécule dithiane greffée sur la CPG est clivé par un traitement à l'acide trichloroacétique à 3% massique dans le dichlorométhane, à température ambiante pendant 120s, libérant une fonction hydroxyle,

Dans une deuxième étape, un synthon porteur d'une fonction phosphoramidite et d'une fonction hydroxyle protégée par un groupement diméthoxytrityle, à une concentration de 0.1 M dans l'acétonitrile est couplé, en présence de tétrazole à une concentration de 0.45 M dans l'acétonitrile, à température ambiante, pendant 30s pour les phosphoramidites A, T, C et G, 360s pour le dithiane phosphoramidite et 60s pour le (dibutylamino)-(diéthanolamino) phénothiazinium phosphoramidite.

Dans une troisième étape, les fonctions hydroxyles qui n'ont pas réagi à l'étape précédente sont bloquées par une solution d'anhydride phénoxyacétique/pyridine/tétrahydrofurane (1:1:8), en présence de méthylimidazole à 16% massique dans le tétrahydrofurane, à température ambiante pendant 20s.

Dans une quatrième étape, le phosphite triester est oxydé en phosphate triester par une solution d'iode à une concentration de 0.02 M dans un mélange eau/pyridine/tétrahydrofurane (1:2:7), à température ambiante pendant 30s.

Les quatre étapes sont répétées autant de fois que nécessite la séquence programmée.

A la fin de la synthèse, l'oligonucléotide est désolidarisé du support par traitement au carbonate de potassium à 0.05 M dans le méthanol anhydre (1mL), à température ambiante pendant 6h. La solution est ensuite neutralisée par ajout de 1.5ml d'acétate de tétraéthylammonium 2 M dans l'eau, puis filtrée sur filtres Amicon 3K (Millipore) pour éliminer les sels et composés issus de la déprotection de l'oligonucléotide.

L'oligonucléotide est purifié par chromatographie liquide à haute performance (CLHP) sur une colonne phase inverse Licrospher RP18. L'élution de l'oligonucléotide marqué par le dérivé phénothiazinium est suivie par absorption visible à 677nm. Les fractions correspondantes sont recueillies et concentrées dans un évaporateur SpeedVac.

Le produit est analysé par spectrométrie de masse MALDI-ToF par cocristallisation dans une matrice d'acide 3-hydroxypicolinique. La masse mesurée (18813 Da) correspond à la masse calculée (18814 Da) ce qui prouve que le dérivé phénothiazinium n'a pas été dégradé dans les conditions de synthèse et de déprotection de l'oligonucléotide.

### 7) Données d'oxydo-réduction

Une comparaison des propriétés oxydo-réductrices du diméthylamino phénothiazinium (bleu de méthylène) et du synthon (dibutylamino)-(diéthanolamino) phénothiazinium incorporé sur un oligonucléotide est effectuée en utilisant une cellule électrochimique constituée d'une électrode de travail en or, d'une contre-électrode en platine et d'une électrode de référence au calomel saturé. La technique utilisée est la voltamétrie cyclique. Le tampon utilisé est un tampon Na₂HPO₄/KH₂PO₄ 20mM KCl 250mM de pH 6.7.

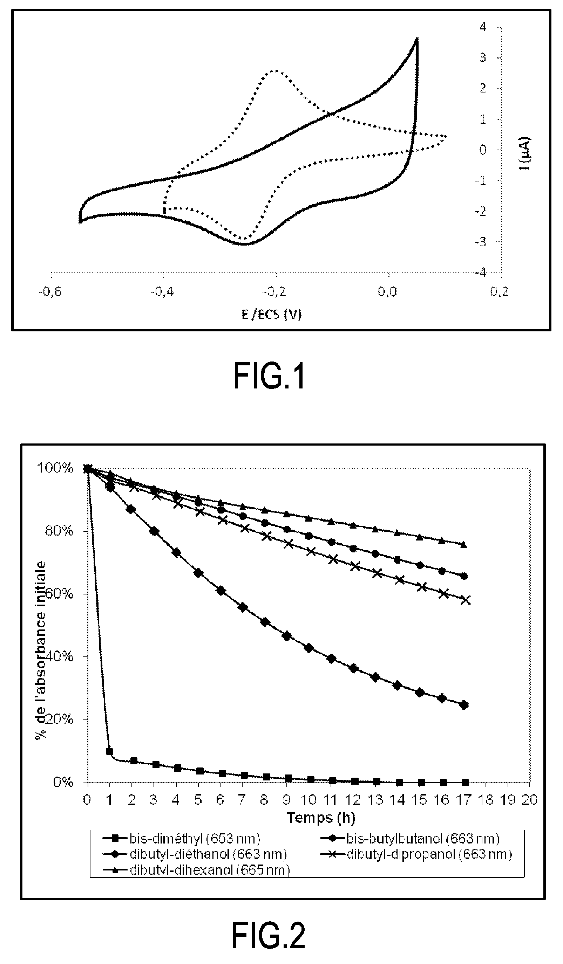

Le bleu de méthylène (courbe en pointillés) est étudié en solution (concentration 15.6µM). Le synthon modifié (courbe en trait plein) est étudié après greffage sur l'électrode d'or d'une solution contenant 2 nmol de l'oligonucléotide marqué. On observe **Figure 1** que les deux courbes montrent des potentiels de réduction et d'oxydation similaires.

### EXEMPLE 2 - SYNTHESE DU DIMETHOXYTRITYL BIS-(BUTYLBUTANOLAMINO) PHENOTHIAZINIUM PHOSPHORAMIDITE

### 1) Préparation de l'iodure de bis-(butylbutanolamino) phénothiazinium

Le tétraiodure de phénothiazinium (145 mg, 0.2 mmol) est dissous dans 4 mL de méthanol à température ambiante. La butylbutanolamine (115 µL, 1 mmol, 5 éq) est ajoutée goutte à goutte à la solution de phénothiazinium, sous agitation magnétique. Le mélange réactionnel est contrôlé par CCM dans un éluant constitué d'un mélange CH₂Cl₂/CH₃OH 9/1 (v/v).

Après 2h de réaction, la solution est concentrée à l'évaporateur rotatif. Le produit est purifié par chromatographie sur gel de silice (éluant CH₂Cl₂/CH₃OH, 95/5, v/v). Une huile pourpre est obtenue avec un rendement de 41% (50 mg, 0.08 mmol).
RMN ¹H (CD₃CN) : δ (ppm) = 7.90-7.23 (m, 6H, H arom.); 3.60 (m, 8H, 4xN-CH₂); 3.00 (m, 4H, 2xCH₂-OH); 1.90-1.55 (m, 12H, 6xCH₂); 1.50-1.32 (m, 4H, 2xCH₂-CH₃); 0.99 (m, 6H, 2xCH₃)
SM (ESI+) : masse calculée = 484.3, masse mesurée = 484.3

### 2) Réparation du iodure de diméthoxytrityl bis-(butylbutanolamino) phénothiazinium phosphoramidite (I.1)

Ce composé est préparé comme à l'exemple 1, 4) et 5).

### EXEMPLE 3 ETUDE DE STABILITE

Il a été démontré qu'en incorporant une dibutylamine et une diéthanolamine ou deux butylbutanolamine, un important gain de stabilité dans une solution de K₂CO₃ (0,05M) dans du méthanol est obtenu par rapport au composé iodure de bis(diméthylamino)phénothiazinium (sel de bleu de méthylène) comme illustré sur la **Figure 2****.**

Le **TABLEAU** ci-dessous met également en évidence les gains de stabilité obtenus avec des amines autres que les diméthylamines présentes sur le bleu de méthylène.

**TABLEAU**

| | | |
|---|---|---|
| 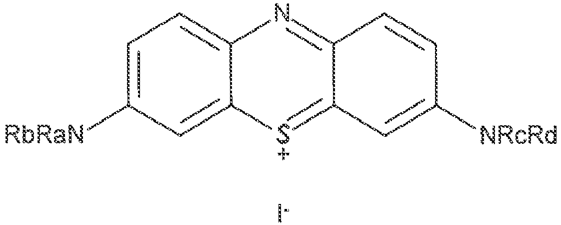 | | |

| Molécules | Stabilité après traitement dans K₂CO₃ 0,05M/MeOH | |
|---|---|---|
| | 4h | 17h |
| Bleu de méthylène Ra=Rb=Rc=Rd= méthyle | 5% | 0% |
| Ra=Rb=Rc=Rd= éthyle | 84% | 67% |
| Ra=Rb=Rc=Rd= (2-éthyl)hexyle | 92% | 74% |
| Ra=Rb=Rc=Rd= butyle | 92% | 78% |
| Ra=Rc=butyle et Rb=Rd=butanol | 91% | 66% |
| Ra=Rb=butyle et Rc=Rd=éthanol | 73% | 25% |
| Ra=Rb=butyle et Rc=Rd=propanol | 89% | 58% |
| Ra=Rb=butyle et Rc=Rd=hexanol | 92% | 76% |

## Revendications

1. Dérivés de diaminophénothiazinium de formule (I) : 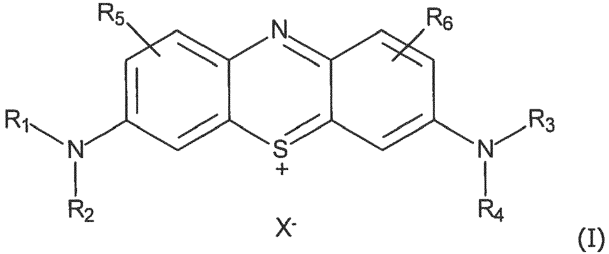 dans laquelle :
- un des groupes R₁, R₂, R₃ et R₄, représente -A₁-OR₇, avec A₁ qui représente une chaîne alkylène linéaire ou ramifiée comprenant de 2 à 12 carbones, les atomes d'oxygène et d'azote étant séparés par au moins deux atomes de carbone consécutifs et R₇ qui représente un groupe phosphoré capable de réagir avec un hydroxyle libre dans des conditions de synthèse des oligonucléotides, ledit groupe formant, avec OA₁ auquel il est lié un groupe phosphoramidite, phosphodiester ou hydrogénophosphonate,
- les autres groupes R₁, R₂, R₃ et R₄, identiques ou différents, représentent indépendamment l'un de l'autre :
o un groupe -A₂-OR₈, avec A₂ qui représente une chaîne alkylène linéaire ou ramifiée comprenant de 2 à 12 carbones, les atomes d'oxygène et d'azote étant séparés par au moins deux atomes de carbone consécutifs, et R₈ qui représente un groupe choisi parmi les groupes trityle, 4-0-monométhoxytrityle, 4,4'-O-diméthoxytrityle, tert-butyldiméthylsilyle, acétyle, trifluoroacétyle, 9-phénylxanthen-9-yle et fluorénylméthyloxycarbonyle,
∘ un groupe alkyle de 2 à 12 atomes de carbone,
∘ ou bien R₁ et R₂ ou R₃ et R₄ sont liés entre eux pour former avec l'atome d'azote auquel ils sont liés un groupe pipéridinyle ou pyrrolidinyle,
- R₅ et R₆ identiques ou différents, représentent, indépendamment l'un de l'autre, un atome d'hydrogène, de chlore, brome, iode ou fluor, ou un groupe alkyle de 1 à 12 atomes de carbone, un groupe alcényle de 2 à 12 atomes de carbone, un groupe alcynyle de 2 à 12 atomes de carbone, un groupe acyle ou un groupe phényle,
- X- représente un anion, de préférence choisi parmi Cl⁻, I⁻, ClO4⁻, NO₃⁻.

2. Dérivés de diaminophénothiazinium selon la revendication 1 **caractérisés en ce que** A₁ et A₂ sont des chaînes alkylène linéaires ou ramifiées, dans lesquelles de 2 à 6 atomes de carbone consécutifs séparent les atomes d'oxygène et d'azote.

3. Dérivés de diaminophénothiazinium selon la revendication 1 ou 2 **caractérisés en ce qu'**au moins un des groupes R₁, R₂, R₃ et R₄, ne représente pas un groupe -A₂-OR₈ tel que défini à la revendication 1 et le ou lesdits groupes R₁, R₂, R₃ ou R₄ différent(s) de -A₁-OR₁ et de -A₂-OR₈ représente(nt) un groupe alkyle de 2 à 12 atomes de carbone, de préférence de 4 à 12 atomes de carbone.

4. Dérivés de diaminophénothiazinium selon l'une des revendications 1 à 3 **caractérisés en ce que** R₅ = R₆ = H.

5. Dérivés de diaminophénothiazinium selon l'une des revendications 1 à 4 **caractérisés en ce que** R₇ représente un groupe -P{N[(C₂-Ci₂)alkyle]₂}(OCH₂CH₂C=N), tel que le groupe -P[N(ⁱPr)₂](OCH₂CH₂C≡N).

6. Dérivés de diaminophénothiazinium selon l'une des revendications précédentes de formule (Ia) : 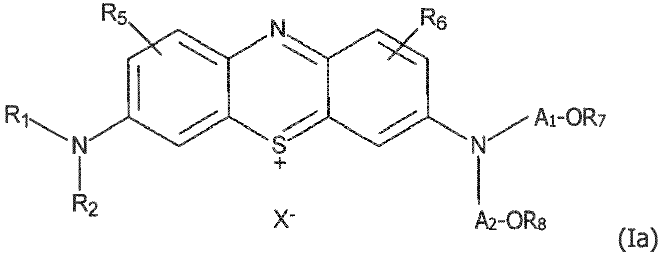 dans laquelle :
- A₁, A₂, R₅, R₆, R₇, R₈ et X⁻ sont tels que définis aux revendications 1, 2, 4 ou 5,
- R₁ et R₂, identiques ou différents, représentent indépendamment l'un de l'autre un groupe alkyle de 2 à 12 atomes de carbone, de préférence de 4 à 12 atomes de carbone, ou R₁ et R₂ sont liés entre eux pour former avec l'atome d'azote auquel ils sont liés un groupe pipéridinyle ou pyrrolidinyle.

7. Dérivés de diaminophénothiazinium selon l'une des revendications 1 à 5 de formule (Ib) : 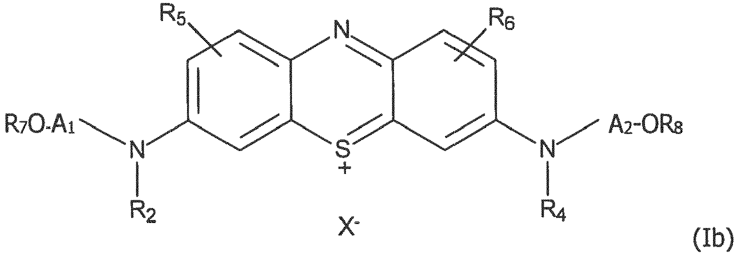 dans laquelle :
- A₁, A₂, R₅, R₆, R₇, R₈ et X⁻ sont tels que définis aux revendications 1, 2, 4 ou 5,
- R₂ et R₄, identiques ou différents, représentent indépendamment l'un de l'autre un groupe alkyle de 2 à 12 atomes de carbone, de préférence de 4 à 12 atomes de carbone.

8. Dérivés de diaminophénothiazinium selon l'une des revendications 1 à 5 de formule (Ic) : 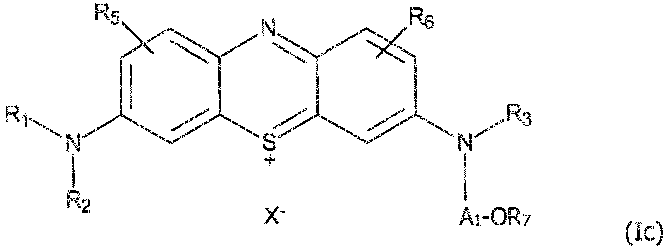 dans laquelle :
- A₁, R₅, R₆, R₇ et X⁻ sont tels que définis aux revendications 1, 2, 4 ou 5,
- R₁ et R₂, identiques ou différents, représentent indépendamment l'un de l'autre un groupe alkyle de 2 à 12 atomes de carbone, de préférence de 4 à 12 atomes de carbone, ou R₁ et R₂ sont liés entre eux pour former avec l'atome d'azote auquel ils sont liés un groupe pipéridinyle ou pyrrolidinyle,
- R₃ représente un groupe alkyle de 2 à 12 atomes de carbone, de préférence de 4 à 12 atomes de carbone.

9. Procédé de marquage d'un oligonucléotide par un dérivé de diaminophénothiazinium selon l'une quelconque des revendications précédentes, qui comprend la croissance d'un oligonucléotide greffé sur un support solide, et le remplacement d'un ou plusieurs des nucléotides le constituant par un ou plusieurs desdits dérivés de diaminophénothiazinium, avant que l'oligonucléotide ne soit détaché du support solide.

10. Procédé de marquage selon la revendication 9 **caractérisé en ce qu'**au moins un remplacement par un dérivé de diaminophénothiazinium est réalisé avant la fin de la croissance de l'oligonucléotide.

11. Procédé de marquage selon la revendication 9 ou 10, **caractérisé en ce qu'**au moins une substitution par un dérivé de diaminophénothiazinium est effectuée en positions 3' ou 5', sur le premier ou le dernier nucléotide, respectivement.

12. Procédé de marquage selon l'une des revendications 9 à 11 **caractérisé en ce qu'**il comprend une étape finale de traitement en milieu basique, soit grâce à une solution basique de soude, d'ammoniaque, ou de carbonate de potassium ou d'une solution de méthylamine et d'ammoniaque ou d'une solution de diisopropylamine et de ß-mercaptoéthanol, soit grâce à de l'ammoniac ou de la méthylamine en phase gazeuse.

13. Support de synthèse d'oligonucléotides, comprenant au moins un dérivé de diaminophénothiazinium greffé de manière covalente en surface selon l'enchainement : 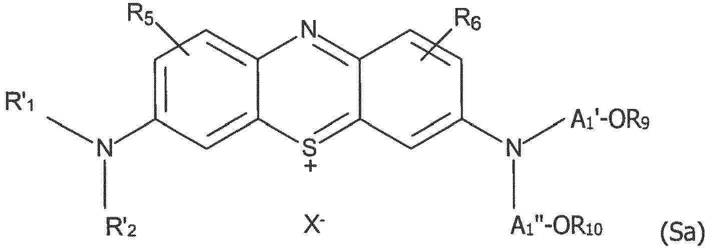 dans laquelle :
- R₅, R₆ et X- sont tels que définis à la revendication 1 ou 4,
- R'₁ et R'₂, identiques ou différents, représentent indépendamment l'un de l'autre un groupe alkyle de 2 à 12 atomes de carbone, de préférence de 4 à 12 atomes de carbone, ou R₁ et R₂ sont liés entre eux pour former avec l'atome d'azote auquel ils sont liés un groupe pipéridinyle ou pyrrolidinyle,
- A₁' et A₁", identiques ou différents, représentent, indépendamment l'un de l'autre, une chaine A₁ telle que définie à la revendication 1 ou 2, et
- R₉ représente un groupe choisi parmi les groupes trityle, 4-O-monométhoxytrityle, 4,4'-O-diméthoxytrityle, tert-butyldiméthylsilyle, acétyle, trifluoroacétyle, 9-phénylxanthen-9-yle et fluorénylméthyloxycarbonyle; et
- R₁₀ représente -CO-A₃-CO-NH-Support, avec A₃ qui représente une chaîne alkylène linéaire ou ramifiée comprenant de 1 à 6 atomes de carbone ; ou 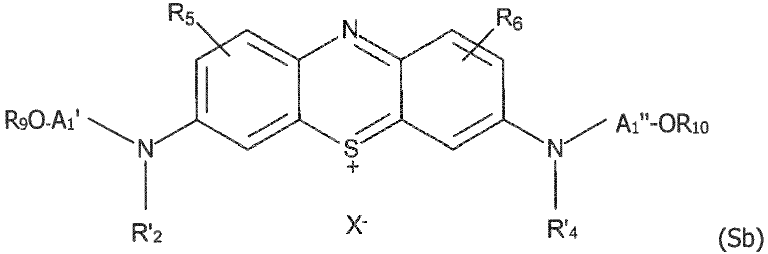
dans laquelle :
- R₅, R₆ et X⁻ sont tels que définis à la revendication 1 ou 4,
- R'₂ et R'₄, identiques ou différents, représentent indépendamment l'un de l'autre un groupe alkyle de 2 à 12 atomes de carbone, de préférence de 4 à 12 atomes de carbone,
- A₁' et A₁", identiques ou différents, représentent, indépendamment l'un de l'autre, une chaine A₁ telle que définie à la revendication 1 ou 2, et
- R₉ représente un groupe choisi parmi les groupes trityle, 4-0-monométhoxytrityle, 4,4'-O-diméthoxytrityle, tert-butyldiméthylsilyle, acétyle, trifluoroacétyle, 9-phénylxanthen-9-yle et fluorénylméthyloxycarbonyle ; et
- R₁₀ représente -CO-A₃-CO-NH-Support, avec A₃ qui représente une chaîne alkylène linéaire ou ramifiée comprenant de 1 à 6 atomes de carbone.

14. Support de synthèse d'oligonucléotides selon la revendication 13 **caractérisé en ce que** le support est choisi parmi les résines, en particulier parmi les résines à base de polystyrène, polyacrylamide, polyéthylèneglycol, cellulose, polyéthylène, polyester, latex, polyamide, polydiméthylacrylamide, les polymères hydrophiles synthétiques ou naturels, les verres et silices à porosité contrôlée, les billes de verre, les gels de silice.

15. Oligonucléotides marqués **caractérisés en ce qu'**ils sont obtenus par le procédé de marquage selon l'une des revendications 9 à 12 ou à partir d'un support solide tel que défini à la revendication 13 ou 14.

## Patentansprüche

1. Diaminophenothiazin-Derivate der Formel (I): 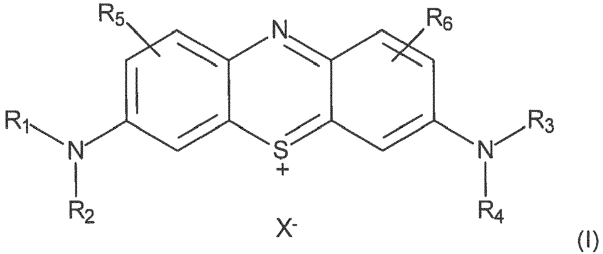 in der:
- eine der Gruppen R₁, R₂, R₃ und R₄ -A₁-OR₇ darstellt, wobei A₁ eine lineare oder verzweigte Alkylenkette mit 2 bis 12 Kohlenstoffen darstellt, wobei die Sauerstoff-und Stickstoffatome durch wenigstens zwei aufeinanderfolgende Kohlenstoffatome getrennt sind, und wobei R₇ eine Phosphorgruppe darstellt, die in der Lage ist, mit einem freien Hydroxyl unter Bedingungen einer Oligonukleotid-Synthese zu reagieren, wobei die Gruppe mit OA₁, an das sie gebunden ist, eine Phosphoramidit-, Phosphodiester- oder Hydrogenphosphonat-Gruppe bildet,
- die anderen Gruppen R₁' R₂, R₃ und R₄, die identisch oder unterschiedlich sind, unabhängig voneinander darstellen:
∘ eine -A₂-OR₈-Gruppe, wobei A₂ eine lineare oder verzweigte Alkylenkette mit 2 bis 12 Kohlenstoffen darstellt, wobei die Sauerstoff- und Stickstoffatome durch wenigstens zwei aufeinanderfolgende Kohlenstoffatome getrennt sind, und wobei R₈ eine Gruppe, die aus den Trityl-, 4-O-Monomethoxytrityl-, 4,4'-O-Dimethoxytrityl-, tert-Butyldimethylsilyl-, Acetyl-, Trifluoracetyl-, 9-Phenylxanthen-9-yl- und Fluorenylmethyloxycarbonyl-Gruppen ausgewählt ist, darstellt,
∘ eine Alkylgruppe mit 2 bis 12 Kohlenstoffatomen, oder aber
∘ R₁ und R₂ oder R₃ und R₄ untereinander verbunden sind, um mit dem Stickstoffatom, an das sie gebunden sind, eine Piperidinyl- oder Pyrrolidinyl-Gruppe zu bilden,
- R₅ und R₆, die identisch oder unterschiedlich sind, unabhängig voneinander ein Wasserstoff-, Chlor-, Brom-, lod- oder Fluoratom oder eine Alkylgruppe mit 1 bis 12 Kohlenstoffatomen, eine Alkenylgruppe mit 2 bis 12 Kohlenstoffatomen, eine Alkynylgruppe mit 2 bis 12 Kohlenstoffatomen, eine Acylgruppe oder eine Phenylgruppe darstellen,
- X⁻ ein Anion, das vorzugsweise aus Cl⁻, I⁻ , ClO₄⁻, NO₃⁻ ausgewählt ist, darstellt.

2. Diaminophenothiazin-Derivate nach Anspruch 1, **dadurch gekennzeichnet, dass** A₁ und A₂ lineare oder verzweigte Alkylenketten sind, bei denen 2 bis 6 aufeinanderfolgende Kohlenstoffatome die Sauerstoff- und Stickstoffatome trennen.

3. Diaminophenothiazin-Derivate nach Anspruch 1 oder 2, **dadurch gekennzeichnet, dass** wenigstens eine der Gruppen R₁, R₂, R₃ und R₄ nicht eine -A₂-OR₈-Gruppe, wie sie in Anspruch 1 definiert ist, darstellt und die Gruppe oder Gruppen R₁, R₂, R₃ oder R₄, die von -A₁-OR₁ und von -A₂-OR₈ verschieden ist/sind, eine Alkylgruppe mit 2 bis 12 Kohlenstoffatomen, vorzugsweise mit 4 bis 12 Kohlenstoffatomen, darstellt/darstellen.

4. Diaminophenothiazin-Derivate nach einem der Ansprüche 1 bis 3, **dadurch gekennzeichnet, dass** R₅ = R₆ = H.

5. Diaminophenothiazin-Derivate nach einem der Ansprüche 1 bis 4, **dadurch gekennzeichnet, dass** R₇ eine -P{N[(C₂-C₁₂)alkyl]₂}(OCH₂CH₂C≡N)-Gruppe, wie die -P[N(ⁱPr)₂](OCH₂CH₂C≡N)-Gruppe darstellt.

6. Diaminophenothiazin-Derivate nach einem der vorhergehenden Ansprüche, der Formel (la): 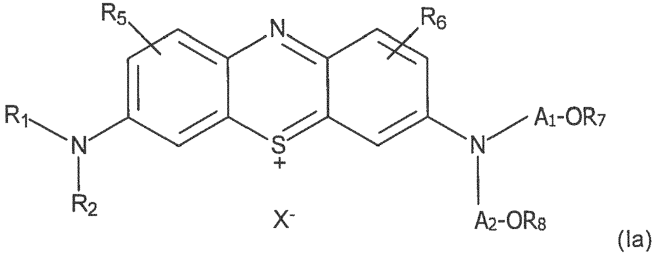 in der:
- A₁, A₂, R₅, R₆ R₇, R₈ und X⁻ derart sind, wie sie in den Ansprüchen 1, 2, 4 oder 5 definiert sind,
- R₁ und R₂, die identisch oder unterschiedlich sind, unabhängig voneinander eine Alkylgruppe mit 2 bis 12 Kohlenstoffatomen, vorzugsweise mit 4 bis 12 Kohlenstoffatomen darstellen oder R₁ und R₂ untereinander verbunden sind, um mit dem Stickstoffatom, an das sie gebunden sind, eine Piperidinyl- oder Pyrrolidinyl-Gruppe zu bilden.

7. Diaminophenothiazin-Derivate nach einem der Ansprüche 1 bis 5, der Formel (Ib): 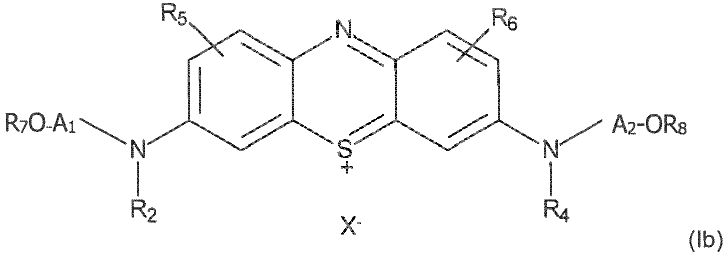 in der:
- A₁, A₂, R₅, R₆ R₇, R₈ und X⁻ derart sind, wie sie in den Ansprüchen 1, 2, 4 oder 5 definiert sind,
- R₂ und R₄, die identisch oder unterschiedlich sind, unabhängig voneinander eine Alkylgruppe mit 2 bis 12 Kohlenstoffatomen, vorzugsweise mit 4 bis 12 Kohlenstoffatomen darstellen.

8. Diaminophenothiazin-Derivate nach einem der Ansprüche 1 bis 5, der Formel (Ic): 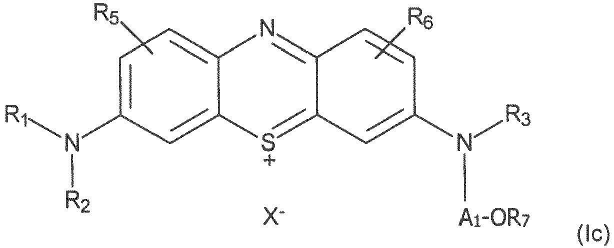 in der:
- A₁, R₅, R₆, R₇ und X⁻ derart sind, wie sie in den Ansprüchen 1, 2, 4 oder 5 definiert sind,
- R₁ und R₂, die identisch oder unterschiedlich sind, unabhängig voneinander eine Alkylgruppe mit 2 bis 12 Kohlenstoffatomen, vorzugsweise mit 4 bis 12 Kohlenstoffatomen darstellen oder R₁ und R₂ untereinander verbunden sind, um mit dem Stickstoffatom, an das sie gebunden sind, eine Piperidinyl- oder Pyrrolidinyl-Gruppe zu bilden.

9. Verfahren zur Markierung eines Oligonukleotids mit einem Diaminophenothiazin-Derivat nach einem der vorhergehenden Ansprüche, das das Wachstum eines auf einen festen Träger gepfropften Oligonukleotids und das Ersetzen von einem oder mehreren der Nukleotide, die es bilden, durch ein oder mehrere der Diaminophenothiazin-Derivate, bevor das Oligonukleotid von dem festen Träger abgelöst wird, umfasst.

10. Markierungsverfahren nach Anspruch 9, **dadurch gekennzeichnet, dass** wenigstens ein Ersetzen durch ein Diaminophenothiazin-Derivat vor dem Ende des Wachstums des Oligonukleotids vollzogen wird.

11. Markierungsverfahren nach Anspruch 9 oder 10, **dadurch gekennzeichnet, dass** wenigstens eine Substitution durch ein Diaminophenothiazin-Derivat in 3'- oder 5'-Stellung an dem ersten bzw. dem letzten Nukleotid vorgenommen wird.

12. Markierungsverfahren nach einem der Ansprüche 9 bis 11, **dadurch gekennzeichnet, dass** es einen abschließenden Schritt zur Behandlung in basischem Medium, entweder dank einer basischen Lösung von Natriumkarbonat, Ammoniakwasser oder Kaliumkarbonat oder einer Lösung von Methylamin und Ammoniakwasser oder einer Lösung von Diisopropylamin und von β-Mercaptoethanol, oder dank Ammoniak oder Methylamin in der Gasphase umfasst.

13. Träger zur Oligonukleotidsynthese, umfassend wenigstens ein an der Oberfläche kovalent gepfropftes Diaminophenothiazin-Derivat, entsprechend der Verkettung: 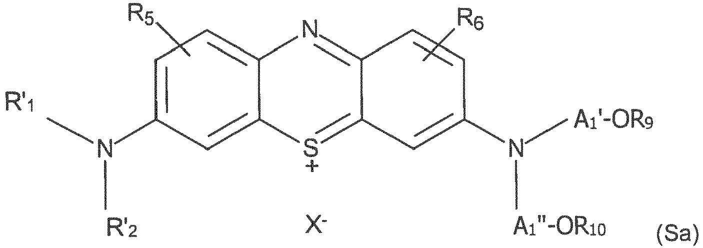 worin:
- R₅, R₆ und X⁻ derart sind, wie sie in Anspruch 1 oder 4 definiert sind,
- R'₁ und R'₂, die identisch oder unterschiedlich sind, unabhängig voneinander eine Alkylgruppe mit 2 bis 12 Kohlenstoffatomen, vorzugsweise mit 4 bis 12 Kohlenstoffatomen darstellen oder R₁ und R₂ untereinander verbunden sind, um mit dem Stickstoffatom, an das sie gebunden sind, eine Piperidinyl- oder Pyrrolidinyl-Gruppe zu bilden,
- A₁' und A₁", die identisch oder unterschiedlich sind, unabhängig voneinander eine A₁-Kette, wie sie in Anspruch 1 oder 2 definiert ist, darstellen, und
- R₉ eine Gruppe, die aus den Trityl-, 4-O-Monomethoxytrityl-, 4,4'-O-Dimethoxytrityl-, tert-Butyldimethylsilyl-, Acetyl-, Trifluoracetyl-, 9-Phenylxanthen-9-yl-und Fluorenylmethyloxycarbonyl-Gruppen ausgewählt ist, darstellt, und
- R₁₀ einen -CO-A₃-CO-NH-Träger darstellt, wobei A₃ eine lineare oder verzweigte Alkylenkette mit 1 bis 6 Kohlenstoffatomen darstellt, oder 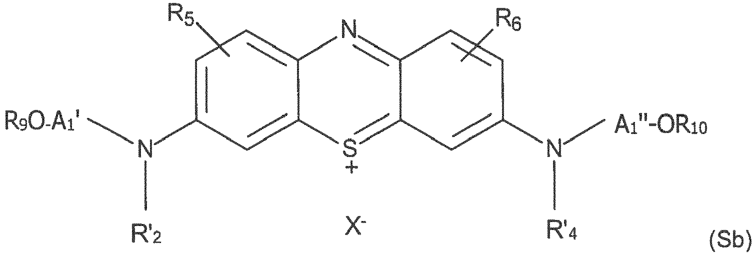
worin:
- R₅, R₆ und X⁻ derart sind, wie sie in Anspruch 1 oder 4 definiert sind,
- R'₂ und R'₄, die identisch oder unterschiedlich sind, unabhängig voneinander eine Alkylgruppe mit 2 bis 12 Kohlenstoffatomen, vorzugsweise mit 4 bis 12 Kohlenstoffatomen darstellen,
- A₁' und A₁", die identisch oder unterschiedlich sind, unabhängig voneinander eine A₁-Kette, wie sie in Anspruch 1 oder 2 definiert ist, darstellen, und
- R₉ eine Gruppe, die aus den Trityl-, 4-O-Monomethoxytrityl-, 4,4'-O-Dimethoxytrityl-, tert-Butyldimethylsilyl-, Acetyl-, Trifluoracetyl-, 9-Phenylxanthen-9-yl-und Fluorenylmethyloxycarbonyl-Gruppen ausgewählt ist, darstellt, und
- R₁₀ einen -CO-A₃-CO-NH-Träger darstellt, wobei A₃ eine lineare oder verzweigte Alkylenkette mit 1 bis 6 Kohlenstoffatomen darstellt.

14. Träger zur Oligonukleotidsynthese nach Anspruch 13, **dadurch gekennzeichnet, dass** der Träger aus Harzen, insbesondere aus Harzen auf Basis von Polystyrol, Polyacrylamid, Polyethylenglycol, Cellulose, Polyethylen, Polyester, Latex, Polyamid, Polydimethylacrylamid, synthetischen oder natürlichen hydrophilen Polymeren, Gläsern und Silika mit kontrollierter Porosität, Glaskugeln, Kieselgelen ausgewählt ist.

15. Markierte Oligonukleotide, **dadurch gekennzeichnet, dass** sie durch das Markierungsverfahren nach einem der Ansprüche 9 bis 12 oder mittels eines festen Trägers, wie er in Anspruch 13 oder 14 definiert ist, erhalten werden.

## Claims

1. Diaminophenothiazinium derivatives of formula (I): 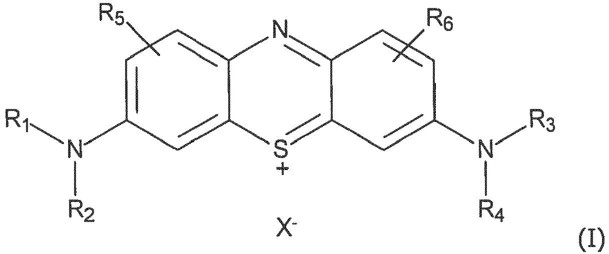 in which:
- one of the R₁, R₂, R₃ and R₄ groups represents -A₁-OR₇, with A₁ representing a linear or branched alkylene chain comprising from 2 to 12 carbons, the oxygen and nitrogen atoms being separated by at least two consecutive carbon atoms, and R₇ representing a phosphorus-comprising group capable of reacting with a free hydroxyl under oligonucleotide synthesis conditions, said group forming, with OA₁ to which it is bonded, a phosphoramidite, phosphodiester or hydrogen phosphonate group,
- the other R₁, R₂, R₃ and R₄ groups, which may be identical or different, represent, independently of one another:
o an -A₂-OR₈ group, with A₂ representing a linear or branched alkylene chain comprising from 2 to 12 carbons, the oxygen and nitrogen atoms being separated by at least two consecutive carbon atoms, and R₈ representing a group chosen from trityl, 4-O-monomethoxytrityl, 4,4'-O-dimethoxytrityl, tert-butyldimethylsilyl, acetyl, trifluoroacetyl, 9-phenylxanthen-9-yl and fluorenylmethyloxycarbonyl groups,
○ an alkyl group having from 2 to 12 carbon atoms,
○ or else R₁ and R₂ or R₃ and R₄ are bonded to one another to form, with the nitrogen atom to which they are bonded, a piperidinyl or pyrrolidinyl group,
- R₅ and R₆, which may be identical or different, represent, independently of one another, a hydrogen, chlorine, bromine, iodine or fluorine atom, or an alkyl group having from 1 to 12 carbon atoms, an alkenyl group having from 2 to 12 carbon atoms, an alkynyl group having from 2 to 12 carbon atoms, an acyl group or a phenyl group,
- X⁻ represents an anion, preferably chosen from Cl⁻, I⁻, ClO₄⁻ and NO₃⁻.

2. The diaminophenothiazinium derivatives as claimed in claim 1, **characterized in that** A₁ and A₂ are linear or branched alkylene chains in which from 2 to 6 consecutive carbon atoms separate the oxygen and nitrogen atoms.

3. The diaminophenothiazinium derivatives as claimed in claim 1 or 2, **characterized in that** at least one of the R₁, R₂, R₃ and R₄ groups does not represent an -A₂-OP₈ group as defined in claim 1 and said R₁, R₂, R₃ or R₄ group(s) different than -A₁-OR₁ and than -A₂-OR₈ represent(s) an alkyl group having from 2 to 12 carbon atoms, preferably from 4 to 12 carbon atoms.

4. The diaminophenothiazinium derivatives as claimed in one of claims 1 to 3, **characterized in that** R₅ = R₆ = H.

5. The diaminophenothiazinium derivatives as claimed in one of claims 1 to 4, **characterized in that** R₇ represents a -P{N[(C₂-C₁₂)alkyl]₂}(OCH₂CH₂C≡N) group, such as the -P[N(ⁱPr)₂](OCH₂CH₂C≡N) group.

6. The diaminophenothiazinium derivatives as claimed in one of the preceding claims, of formula (Ia): 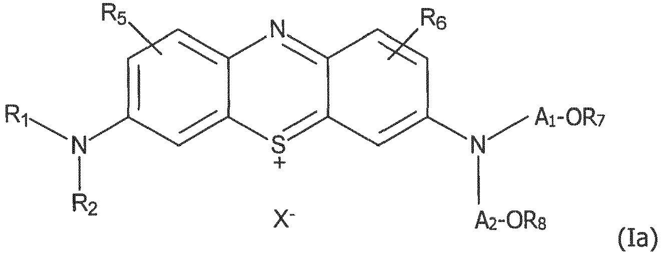 in which:
- A₁, A₂, R₅, R₆, R₇, R₈ and X⁻ are as defined in claim 1, 2, 4 or 5,
- R₁ and R₂, which may be identical or different, represent, independently of one another, an alkyl group having from 2 to 12 carbon atoms, preferably from 4 to 12 carbon atoms, or R₁ and R₂ are bonded to one another to form, with the nitrogen atom to which they are bonded, a piperidinyl or pyrrolidinyl group.

7. The diaminophenothiazinium derivatives as claimed in one of claims 1 to 5, of formula (Ib): 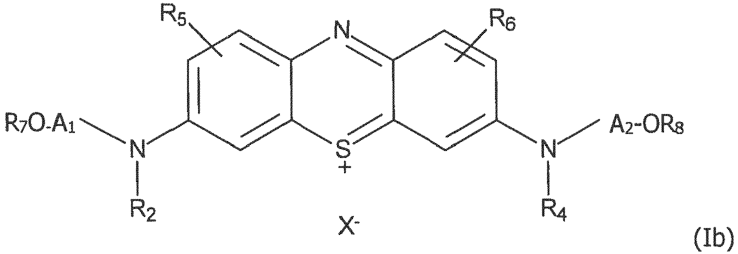 in which:
- A₁, A₂, R₅, R₆, R₇, R₈ and X⁻ are as defined in claim 1, 2, 4 or 5,
- R₂ and R₄, which may be identical or different, represent, independently of one another, an alkyl group having from 2 to 12 carbon atoms, preferably from 4 to 12 carbon atoms.

8. The diaminophenothiazinium derivatives as claimed in one of claims 1 to 5, of formula (Ic): 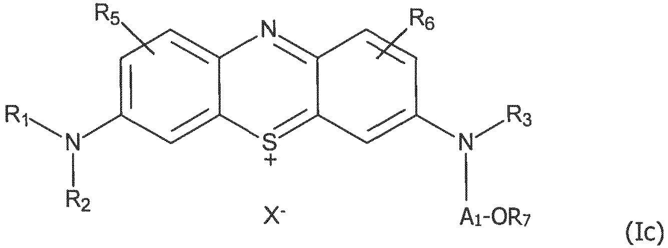 in which:
- A₁, R₅, R₆, R₇ and X⁻ are as defined in claim 1, 2, 4 or 5,
- R₁ and R₂, which may be identical or different, represent, independently of one another, an alkyl group having from 2 to 12 carbon atoms, preferably from 4 to 12 carbon atoms, or R₁ and R₂ are bonded to one another to form, with the nitrogen atom to which they are bonded, a piperidinyl or pyrrolidinyl group,
- R₃ represents an alkyl group having from 2 to 12 carbon atoms, preferably from 4 to 12 carbon atoms.

9. A method for labeling an oligonucleotide with a diaminophenothiazinium derivative as claimed in any one of the preceding claims, which comprises the growth of an oligonucleotide grafted onto a solid substrate, and the replacement of one or more of the nucleotides of which it is formed with one or more of said diaminophenothiazinium derivatives, before the oligonucleotide is detached from the solid substrate.

10. The labeling method as claimed in claim 9, **characterized in that** at least one replacement with a diaminophenothiazinium derivative is carried out before the end of the growth of the oligonucleotide.

11. The labeling method as claimed in claim 9 or 10, **characterized in that** at least one substitution with a diaminophenothiazinium derivative is carried out in the 3' or 5' positions, on the first or the last nucleotide, respectively.

12. The labeling method as claimed in one of claims 9 to 11, **characterized in that** it comprises a final step of treatment in a basic medium, either using a basic solution of sodium hydroxide, of aqueous ammonia or of potassium carbonate or a solution of methylamine and of aqueous ammonia or a solution of diisopropylamine and of ß-mercaptoethanol, or using ammonia or methylamine in the gas phase.

13. An oligonucleotide synthesis substrate comprising at least one diaminophenothiazinium derivative covalently grafted at the surface according to the series: 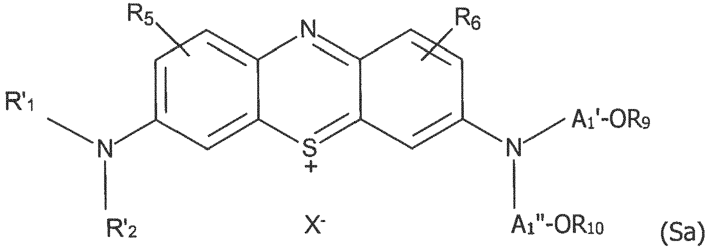 in which:
- R₅, R₆ and X⁻ are as defined in claim 1 or 4,
- R'₁ and R'₂, which may be identical or different, represent, independently of one another, an alkyl group having from 2 to 12 carbon atoms, preferably from 4 to 12 carbon atoms, or R₁ and R₂ are bonded to one another to form, with the nitrogen atom to which they are bonded, a piperidinyl or pyrrolidinyl group,
- A₁' and A₁", which may be identical or different, represent, independently of one another, an A₁ chain as defined in claim 1 or 2, and
- R₉ represents a group chosen from trityl, 4-O-monomethoxytrityl, 4,4'-O-dimethoxytrityl, tert-butyldimethylsilyl, acetyl, trifluoroacetyl, 9-phenylxanthen-9-yl and fluorenylmethyloxycarbonyl groups; and
- R₁₀ represents -CO-A₃-CO-NH-Substrate, with A₃ representing a linear or branched alkylene chain comprising from 1 to 6 carbon atoms; or 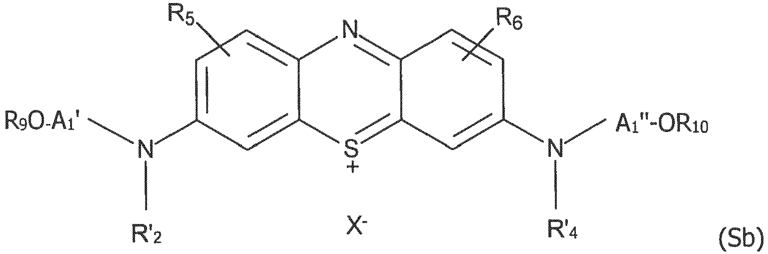
in which:
- R₅, R₆ and X⁻ are as defined in claim 1 or 4,
- R'₂ and R'₄, which may be identical or different, represent, independently of one another, an alkyl group having from 2 to 12 carbon atoms, preferably from 4 to 12 carbon atoms,
- A₁' and A₁", which may be identical or different, represent, independently of one another, an A₁ chain as defined in claim 1 or 2, and
- R₉ represents a group chosen from trityl, 4-O-monomethoxytrityl, 4,4'-0-dimethoxytrityl, tert-butyldimethylsilyl, acetyl, trifluoroacetyl, 9-phenylxanthen-9-yl and fluorenylmethyloxycarbonyl groups; and
- R₁₀ represents -CO-A₃-CO-NH-Substrate, with A₃ representing a linear or branched alkylene chain comprising from 1 to 6 carbon atoms.

14. The oligonucleotide synthesis substrate as claimed in claim 13, **characterized in that** the substrate is chosen from resins, in particular from resins based on polystyrene, polyacrylamide, polyethylene glycol, cellulose, polyethylene, polyester, latex, polyamide, polydimethylacrylamide, synthetic or natural hydrophilic polymers, glasses and silicas with a controlled porosity, glass beads or silica gels.

15. Labeled oligonucleotides **characterized in that** they are obtained by means of the labeling method as claimed in one of claims 9 to 12 or from a solid substrate as defined in claim 13 or 14.
